(19) Europäisches Patentamt / European Patent Office / Office européen des brevets

(11) **EP 4 406 547 A1**

(12) **EUROPEAN PATENT APPLICATION**
published in accordance with Art. 153(4) EPC

(43) Date of publication:
**31.07.2024  Bulletin 2024/31**

(21) Application number: 22871715.3

(22) Date of filing: **22.08.2022**

(51) International Patent Classification (IPC):
**A61K 38/17** (2006.01)    **A61K 47/64** (2017.01)
**A61K 47/68** (2017.01)    **A61P 25/22** (2006.01)
**A61P 25/24** (2006.01)    **C07K 14/705** (2006.01)
**C12N 15/12** (2006.01)

(86) International application number:
**PCT/CN2022/113892**

(87) International publication number:
**WO 2023/045662 (30.03.2023 Gazette 2023/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.09.2021  CN 202111128332**

(71) Applicant: **Shenzhen Chenyang Biological
Technology Co., Ltd
Shenzhen, Guangdong 518000 (CN)**

(72) Inventors:
• **TAN, Zhen
  Shenzhen, Guangdong 518000 (CN)**
• **YU, Zhijian
  Shenzhen, Guangdong 518000 (CN)**
• **LI, Shupeng
  Shenzhen, Guangdong 518000 (CN)**

(74) Representative: **Kailuweit & Uhlemann
Patentanwälte
Partnerschaft mbB
Bamberger Straße 49
01187 Dresden (DE)**

Remarks:
The complete document including Reference
Table(s) and the Sequence Listing(s) can be
downloaded from the EPO website

(54) **ANTI-DEPRESSION AND ANTI-ANXIETY APPLICATION OF POLYPEPTIDE AND COMPLEX**

(57)    The present invention relates to anti-depression and anti-anxiety application of a polypeptide, and provides application of a polypeptide having a sequence as shown in SEQ ID NO: 1 in preparation of a medicine for treating and/or preventing depression or anxiety. The present invention further relates to a complex containing the polypeptide.

**FIG. 7 is the Figure Accompanying the Abstract**

EP 4 406 547 A1

**Description**

**Field of the Invention**

The invention relates to the field of anti-depression and anti-anxiety, in particular to the use of a polypeptide in anti-depression and anti-anxiety.

**Background of the Invention**

Depression is a mental disorder disease caused by various reasons, with a low mood as its main clinical symptom. It has the characteristics of high morbidity, high disability rate, high suicide rate and the like. The pathogenesis of depression has not been fully clarified, and most of the antidepressants commonly used in clinical practice are developed based on the "monoamine neurotransmitter hypothesis". This hypothesis suggests that the occurrence of depression is related to the deficiency of monoamine neurotransmitters such as Dopamine (DA), 5-hydroxytryptamine (5-HT) and norepinephrine (NA) in the brain, and that antidepressants block the reuptake of monoamine neurotransmitters by inhibiting the function of 5-HT and NA transporters, and increase their concentration in synaptic space, thereby improving depression symptoms.

According to the mechanism of action and the development time thereof, antidepressants commonly used in clinical practice are divided into the following categories, namely, the first generation of antidepressants: monoamine oxidase inhibitors (MAOI) and tricyclic antidepressants; the second generation of antidepressants: selective serotonin reuptake inhibitor (SSRI); the third generation of antidepressants: dual reuptake inhibitors of 5-hydroxytryptamine and norepinephrine, norepinephrine reuptake inhibitors (SNRI), norepinephrine-related and serotoninergic antidepressants, etc.

Although these drugs developed according to the "monoamine neurotransmitter hypothesis" have a clear therapeutic effect, the effective rate is only 60%, and their anti-depression effect is slow to appear (usually 2-3 weeks after taking the drug). In addition, these drugs have some shortcomings such as large adverse reactions during long-term application and easy recurrence after drug discontinuation.

In addition to the classic "monoamine neurotransmitter hypothesis" of depression, in recent years, with the in-depth study of the pathogenesis of depression, researchers have discovered some new molecular targets of antidepressants which are based on non-monoamine transmitters, such as NMDA receptors, CRF1 receptors, $\delta$ receptors, $\kappa$ receptors, GABAB receptors, M choline receptors, IDO, CysLTIR, PDE4, PPAR $\gamma$, PPAR $\delta$, NOS and so on. Some substances with anti-depression activity have been found around these new targets, and some of them have entered the clinical research stage.

In order to provide more abundant and better treatment strategies and better elucidate the pathogenesis, there is still a strong demand to explore and discover more important pathogenesis and therapeutic targets in the field of depression.

**Summary of the Invention**

In order to achieve the above purpose, the inventors have carried out a large number of studies based on the molecular changes of the depression model, and discovered a new mechanism for the pathogenesis of depression, thereby completing the present invention.

Specifically, 5-HT receptors are a group of G protein-coupled receptors and ligand-gated ion channels that appear at the center of the central nervous system as well as around the peripheral nervous system. 5HT receptors can be divided into seven subfamilies: 5-HT1, 5-HT2, 5-HT3, 5-HT4, 5-HT5, 5-HT6 and 5-HT7. 5-HT2 receptor has three subtypes: A, B and C, namely, 5-HT2A, 5-HT2B and 5-HT2C receptor proteins. Among them, the 5-HT2A receptor (5-HT2AR), as an excitatory 5-HT receptor, is widely expressed in mammalian brain.

DA receptor is a kind of receptor located in an organism which acts by means of its corresponding membrane receptor. DA receptors can be divided into five types: D1, D2, D3, D4 and D5. D1 receptor (D1R) is widely expressed in brain.

The inventors recognize that 5-HT2AR, in addition to its individual form, also regulates neurotransmitters through a complex, such as 5-HT1AR/5-HT2AR and 5-HT2AR/oxytocin receptor (OXTR).

At present, there are no reports showing that 5-HT2AR can form protein complex with D1R in vivo, much less reports revealing the relevance between 5-HT2AR/D1R complex and depression. Whereas the present inventors unexpectedly discovered that 5-HT2AR, via its carboxyl terminal, forms a protein complex with D1R, and this complex has depressive pathogenicity. Subsequently, through further research, it was found that a polypeptide can reduce the interaction between 5-HT2AR/D1R and thus alleviate depressive and anxiety symptoms.

Accordingly, in a first aspect, the present invention provides a polypeptide having an amino acid sequence as set forth in SEQ ID NO: 1 (SKDNSDGVNEKVSCV). Also provided is the use of the polypeptide of the present invention in the preparation of a medicament for treating and/or preventing depression or anxiety disorder.

In some embodiments, the length of the polypeptide of the present invention may be 15 to 90aa, in particular 15 to 50aa, 15 to 30aa, 15 to 20aa. For example, 16aa, 17aa, 18aa, 19aa, 20aa, 21aa, 22aa, 23aa, 24aa, 25aa, 26aa, 27aa, 28aa, 29aa, 30aa, 31aa, 32aa, 33aa, 34aa, 34aa, 35aa, 36aa, 37aa, 38aa, 39aa, 40aa, 41aa, 42aa, 43aa, 44aa, 45aa, 46aa, 47aa, 48aa or 49aa.

In some embodiments, the polypeptide of the present invention may be derived from 5-HT2AR. The 5-HT2AR from which the polypeptide is derived can be queried from a biological information database (such as Genbank, EMI, DDBJ, etc.), based on which the polypeptide derived from the 5-HT2AR can be confirmed. For example, in the case where the polypeptide is derived from 5-HT2AR (Genbank Accession No.: NP_001365853), in addition to containing the sequence as set forth in SEQ ID NO: 1 (i.e., positions 457 to 471 of the amino acid sequence in NP_001365853), the polypeptide may further include one or more amino acid residues in the N-terminal direction.

In an exemplary embodiment, the polypeptide of the present invention consists of positions 442 to 471 of the amino acid sequence corresponding to NP_001365853.

In an exemplary embodiment, the polypeptide of the present invention consists of positions 385 to 471 of the amino acid sequence corresponding to NP_001365853.

In some embodiments, 5-HT2AR is derived from primate.

In a preferred embodiment, 5-HT2AR is derived from human.

As used herein, the term "treat" refers to causing a desired or beneficial effect in a patient, which may include reducing the frequency or severity of one or more symptoms of a disease, or hindering or inhibiting further development of a disease, illness or disorder.

As used herein, the term "prevent" means avoiding or delaying the onset of a disease or avoiding the onset of its clinical or subclinical symptoms.

The polypeptide of the present invention has a function of anti-depression through a new mechanism (5-HT2AR/D1R complex) in depression pathology, which has the characteristics of quick effect, good activity and little side effect, and has a good clinical development value. In addition, the polypeptide of the present invention can also achieve anti-anxiety effect.

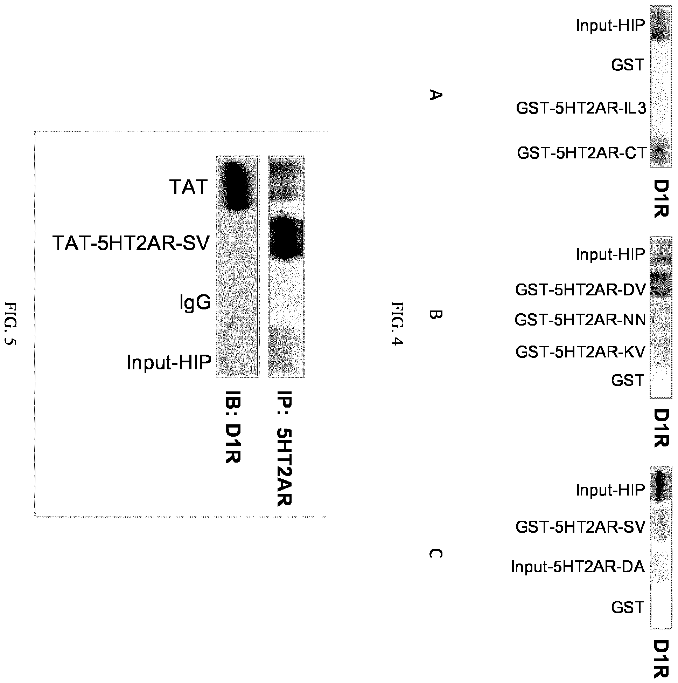

It should be noted that as confirmed in Examples 4 and 7, and FIGs. 5, 6, 9 and 10 below, the polypeptide of the present invention disrupts the interaction of the 5-HT2AR/D1R complex, but does not significantly affect the expression of 5-HT2AR or D1R. In other words, the polypeptide of the present invention functions by specifically disrupting the interaction of the 5-HT2AR/D1R complex.

In a second aspect, the present invention provides a nucleic acid molecule encoding a polypeptide of the present invention. In addition, also provided is the use of the nucleic acid molecule of the present invention in the preparation of a medicament for treating and/or preventing depression or anxiety disorder.

The nucleic acid molecule of the present invention is used to produce the antigenic peptides of the present invention, and the sequence of the nucleic acid molecule can be appropriately adjusted by those skilled in the art according to the expression system employed.

In an exemplary embodiment, the nucleic acid molecule has a sequence as set forth in SEQ ID NO: 2.

In a third aspect, the present invention provides an expression vector comprising the nucleic acid molecule of the present invention. Also provided is the use of the expression vector of the present invention in the preparation of a medicament for treating and/or preventing depression or anxiety disorder.

The nucleic acid sequence of the present invention can be inserted into an expression vector using a variety of known methods. For example, the nucleic acid molecule can be inserted into an appropriate restriction endonuclease site. Standard techniques for cloning, isolation, amplification and purification, and enzymatic reactions involving a DNA ligase, DNA polymerase, restriction endonuclease and various separation techniques in the operation belong to techniques known and commonly used by those skilled in the art.

In a fourth aspect, the present invention provides a host cell comprising the nucleic acid molecule or the expression vector of the present invention. Also provided is the use of the host cell of the present invention in the preparation of a medicament for treating and/or preventing depression or anxiety disorder.

The polypeptide of the present invention may be produced using expression vectors and host cells in a variety of expression systems such as prokaryotic and eukaryotic expression systems. Next, the mammalian expression system is illustrated as an instance. The host cell may include COS-7 cell line of monkey kidney fibroblasts and other cell lines capable of expressing compatible vectors, such as C127, 3T3, CHO, Hela and BHK cell lines. A mammalian expression vector should comprise a replication origin, a suitable promoter and enhancer, and any necessary ribosome binding site, polyadenylation site, splicing donor and receptor site, transcription termination sequence, and 5' flanking non-transcriptional sequence. DNA sequences derived from, for example, SV40 splicing and polyadenylation sites can be used to provide a desired non-transcriptional genetic element. An expression vector may be introduced into a host cell by a variety of methods familiar to those skilled in the art, including but not limited to e.g. calcium phosphate transfection, DEAE-glucan mediated transfection or electroporation.

In a fifth aspect, the present invention provides a complex comprising the polypeptide of the present invention and a (transferring) carrier attached thereto for permeating the blood-brain barrier.

In an exemplary embodiment, the carrier used to permeate the blood-brain barrier may be one or more of HIV-1Tat protein, insulin, cationized albumin, monoclonal antibody against rat transferrin receptor (OX26), mouse-derived monoclonal antibody against human insulin receptor (HIRMAb), Penetratin, transduction domain of Tat protein, Pep-1 peptide, $S4_{13}$-PV, Magainin 2 and Buforin 2. For example, TAT transduction domain, with an amino acid of YGRKKRRQRRR (as set forth in SEQ ID NO: 3), can transduce across the membrane into a cell.

The polypeptide of the present invention can be attached to a carrier for permeating the blood-brain barrier by appropriate linking techniques. Exemplary linking techniques may be avidin-biotin techniques, polyethylene glycol (PEG)-based space arm techniques, fusion protein techniques and the like. For example, in the case of using the transduction domain of HIV-1Tat protein as a carrier for permeating the blood-brain barrier, the polypeptide of the present invention can be directly linked to the transduction domain of Tat protein by fusion protein techniques.

In some embodiments, where fusion protein techniques are used, the polypeptide of the present invention can also be linked to a carrier for permeating the blood-brain barrier using a linker. Exemplary linkers may be flexible linkers with glycine, such as G, GSG, GSGGSG, GSGGSGG, GSGGSGGG, GGGGSGGG, GGGGS and SGG, etc.

In a sixth aspect, the present invention provides a method of treating depression in a subject suffering from depression or being at risk of depression, the method including:
administering an effective amount of the polypeptide or complex of the present invention to the subject.

The polypeptide or complex reduces the 5-HT2AR/D1R interaction, thereby treating depression in the subject.

In addition, the present invention provides a method of treating anxiety disorder in a subject suffering from anxiety disorder or being at risk of anxiety disorder, the method including:
administering an effective amount of the polypeptide or complex of the present invention to the subject.

The polypeptide or complex reduces the 5-HT2AR/D1R interaction, thereby treating anxiety disorder in the subject.

The term "effective amount" or "therapeutically effective amount" refers to an amount of an active agent sufficient to induce a desired biological result. The result may be a reduction in the signs, symptoms or causes of a disease, or any other desired change of a biological system. The term "therapeutically effective amount" is used herein to mean any amount of a formulation that causes a substantial improvement in the condition of the disease when repeatedly administered to the affected area over a period of time. This amount will vary depending on the condition being treated, the stage of progression of the condition, and the type and concentration of the formulation used. Those skilled in the art can determine the appropriate amount by routine experiments.

"Subject", "individual" or "patient" are used interchangeably herein and refer to a vertebrate, preferably a mammal, more preferably a human. Mammals include, but are not limited to, rats, monkeys, humans, farm animals, and pets. Tissues and cells of a biological entity and the descendants thereof which are obtained or cultured in vitro are also included.

In a seventh aspect, the present invention provides the polypeptide or the complex of the present invention, which is used as a drug.

In some embodiments, being used as a drug means being used to treat or prevent depression or anxiety disorder.

## Brief Description of the Drawings

FIG. 1 shows the Western blot results of co-immunoprecipitation of hippocampus tissue from mice;

FIG. 2 shows Western blot results of co-immunoprecipitation from CRS, CMS, and CSDS mouse models;

FIG. 3 shows the positions of the fragments involved in Example 3 on the 5-HT2AR;

FIG. 4 shows the Western blot results of GST pulldown analysis of mouse hippocampus tissue using the fragment of Example 3;

FIG. 5 shows the Western blot results of co-immunoprecipitation of hippocampus tissue from polypeptide-treated mice;

FIG. 6 shows the Western blot results of hippocampus tissue from polypeptide-treated mice;

FIG. 7 shows the results of OFT, FST, and TST analysis of polypeptide-treated mice, wherein *: P < 0.05; **: P < 0.01;

FIG. 8 shows the results of FST, TST, and SPT analysis of polypeptide-treated CRS, CMS, and CSDS mice, wherein *: P < 0.05; **: P < 0.01; ***: P < 0.001; ****: P < 0.0001;

FIG. 9 shows the Western blot results of co-immunoprecipitation of hippocampus tissue from polypeptide-treated CRS, CMS, and CSDS mice;

FIG. 10 shows the Western blot results of hippocampus tissue from polypeptide-treated CRS mice;

FIG. 11 shows the results of OFT (for central region residence time) analysis of polypeptide-treated mice, wherein;

**: P < 0.01;

FIG. 12 shows the results of EPM analysis of polypeptide-treated mice, wherein *: P < 0.05; **: P < 0.01.

## Detailed Description of the Invention

Throughout the specification, terms used herein shall be understood to have the meanings commonly used in the art unless otherwise specifically stated. Accordingly, unless otherwise defined, all technical and scientific terms used herein have the same meaning as generally understood by those skilled in the art which the present invention belongs to. In case of any contradiction, the present application shall take precedence.

Embodiments of the present invention will be described in detail below in combination with the Examples, and the advantages and various effects of the present invention will be more clearly presented therefrom. It shall be understood by those skilled in the art that these specific embodiments and Examples are intended to illustrate but not limit the present invention.

If the specific condition is not specified in an Example, a conventional condition or the condition recommended by the manufacturer shall be followed. If the manufacturer of a reagent or instrument used is not indicated, it is a conventional product available commercially.

Unless otherwise noted, the experimental animals used in the Examples were adult C57BL/6J male mice (12-14 weeks) raised at 18-22°C with 12 hours of illumination/12 hours of darkness. Free access to food and tap water throughout the process.

The statistical analysis involved in the Examples employed a one-way variance (anova) analysis followed by a post-comparison to evaluate the difference between the averages. Unless otherwise stated, an independent sample t-test was used to compare the difference between any two given groups.

Example 1. Confirmation of formation of 5-HT2AR/D1R complex in vivo

Co-immunoprecipitation was performed using protein samples (100-500 $\mu$g protein) from mouse hippocampus tissue. Precipitation was performed using a mouse antibody against 5-HT2AR (SantaCruz Biotechnology, sc-166775) and 25 $\mu$l protein A/G plus agarose bead slurry (santaCruz Biotechnology, sc-2001).

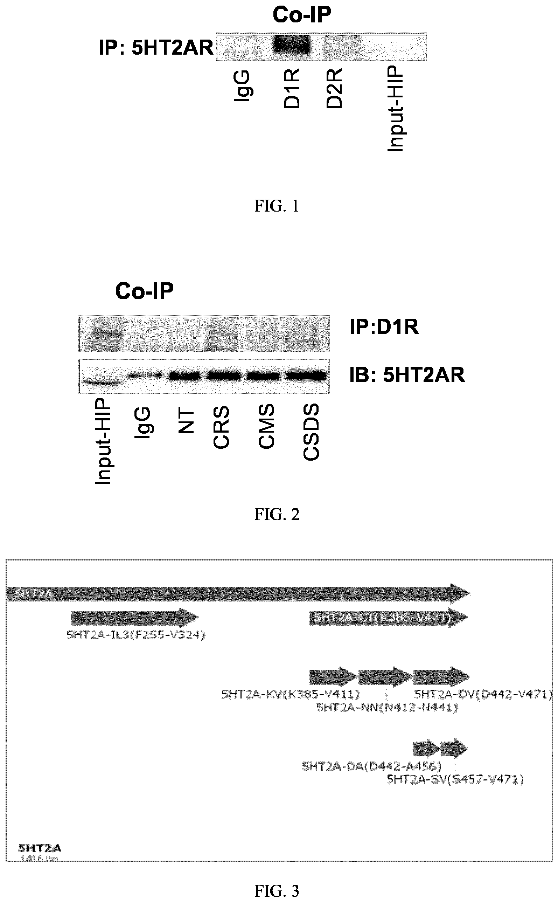

Western blot analysis was carried out after precipitation: the denatured protein was separated on 8% SDS-PAGE gel, then transferred to cellulose nitrate membrane and blocked with TBST; then incubated with mouse antibodies against D1R (SantaCruz Biotechnology, sc-33660) and D2R (SantaCruz Biotechnology, sc-5303) overnight at 4 °C; finally, treated with HRP-coupled secondary antibody for 1 hour, SuperSignal ECL Chemiluminescence Kit was used to detect the signal, and Image Lab software was used to analyze the density of the bands. The results are shown in FIG. 1.

It can be seen from FIG. 1 that anti-5-HT2AR antibody can precipitate with D1R but not D2R, which indicates that 5-HT2AR can form a complex with D1R but not D2R in vivo.

Example 2. Verification of pathological significance of the complex

In order to explore the pathological significance of the complex, we constructed different depression models, including chronic restraint stress (CRS), chronic mild stress (CMS) and chronic social defeat stress (CSDS). The methods for constructing each model are as follows:

Construction of CRS mouse model: Mice were fixed horizontally for 6 hours (10:00 to 16:00) per day in an acrylic cylindrical flat-bottomed restrainer (25 × 90mm) for 2 weeks. The filter has several slots to firmly restrain the mice according to the size of each one, and to inhibit physical movement of their limbs without causing pain. After being restrained, the mice were immediately put back into their cage. Unrestrained mice (control) remained in their home cage in the absence of the CRS procedure, and neither of control mice nor CRS mice could get food and water during exposure to CRS.

Construction of CMS mouse model: Mice were subjected to various pressures, including: restraint (4 hours), cage tilt (45 times, 12 hours each), light-dark cycle reversal (once), flash (12 hours) and dirty cage (2 times, 14 hours each). The program lasted for six weeks. Control mice were not subjected to corresponding pressure.

Construction of CSDS mouse model: Mice were exposed to different CD1 aggressive mice (target mice) for 10 min per day for 10 days. After exposure for 10 min, experimental mice were separated by a plastic separator with holes. In addition, experimental mice were exposed to chronic pressure for 24 hours through a separator placed in the middle of the cage. A similar procedure (non-social stress) was used for the control mice, but with the same breed of mice instead of aggressive mice.

The interaction of D1R and 5-HT2AR in the models are studied according to the co-immunoprecipitation and Western blot methods in Example 1, using the constructed CRS mouse model, CMS mouse model and CSDS mouse model respectively. The results are shown in FIG. 2.

As can be seen from FIG. 2, a significant increase of 5-HT2AR/D1R complex was found in CRS, CMS and CSDS models, which confirms the pathogenetic role of the complex in depression.

Example 3. Identification of complex interbinding site

In order to confirm the interbinding site of the 5-HT2AR/D1R complex, the 5-HT2AR full-length cDNA clone (Genbank Accession No.: NM_001378924) was first amplified to obtain cDNA fragments of the CT region of 5-HT2AR (K385-V471) and the third intracellular loop (IL3) region of 5-HT2AR (F255-V324). These fragments were subcloned to the BamH1/EcoR1 or BamH1/Xho1 site of a pGEX-4T-3 plasmid (YouBio no: VT1255). The initial methionine residue and termination codon were incorporated as appropriate. All constructs were resequenced to confirm that splicing and fusion was achieved appropriately. The GST fusion protein containing the third intracellular loop (IL3) of 5-HT2AR (GST-5HT2AR-IL3) and the GST fusion protein containing the CT region of 5-HT2AR (GST-5HT2AR-CT) were expressed from *E. coli* BL21 viable cells (AlpalifeBio, no: KTSM104L) and purified from bacterial lysate solution. Wherein the specific positions of the coding sequences of IL3 region and CT region on 5-HT2AR are shown in FIG. 3.

500 μg of dissolved mouse hippocampus tissue extract was diluted with 1 × PBS/1% Triton X-100 and then incubated overnight with 20 μl protein GST resin saturated GST protein or 15 μg GST fusion protein at 4 °C. Beads were washed 1-8 times with 1 × PBS/1% Triton X-100. Binding proteins were eluted with 2 × loading buffer, separated by SDS-PAGE, and underwent Western blot with respective antibodies thereof. The results are shown in FIG. 4A.

As can be seen from FIG. 4 A, the CT region of 5-HT2AR can pulldown D1R.

Next, in order to further explore the correct interaction sequence/site between 5-HT2AR and D1R, we divided the CT region into KV (K385-V411) region of 5-HT2AR, NN (N412-N441) region of 5-HT2AR and DV (D442-V471) region of 5-HT2AR. The specific positions of these regions on 5-HT2AR are shown in FIG. 3.

According to the method in the present Example, a pulldown analysis was performed using a GST fusion protein containing a KV region of 5-HT2AR (GST-5HT2AR-KV), a GST fusion protein containing a NN region of 5-HT2AR (GST-5HT2AR-NN), and a GST fusion protein containing a DV region of 5-HT2AR (GST-5HT2AR-DV), and the results of Western blot were shown in FIG. 4 B.

As can be seen from FIG. 4 B, the DV region of 5-HT2AR can pulldown D1R.

Further, we divided the DV region into DA (D442-A456) region of 5-HT2AR and SV (D442-V471) region of 5-HT2AR. The specific positions of these regions on 5-HT2AR are shown in FIG. 3.

According to the method in the present Example, a pulldown analysis was performed using a GST fusion protein containing a DA region of 5-HT2AR (GST-5HT2AR-DA), and a GST fusion protein containing a SV region of 5-HT2AR (GST-5HT2AR-SV), and the results of Western blot were shown in FIG. 4 C.

It can be seen from FIG. 4 C that 5HT2AR-SV polypeptide (S457-V471) has an affinity to D1R from mouse hippocampus tissue, indicating that 5-HT2AR can interact with D1R via carboxyl tail.

Example 4. Confirmation of the destructive effect of the polypeptide on the complex in vivo

In order to verify the effect of 5-HT2AR carboxyl terminal polypeptide on 5-HT2AR/D1R complex in vivo, the C terminal of SV region (S457-V471) of 5-HT2AR was fused with the N terminal of transduction domain of HIV-1-type Tat protein (as shown in SEQ ID NO: 3, hereinafter referred to as TAT) to obtain a fusion protein which can permeate the blood-brain barrier, named TAT-5HT2AR-SV Similarly, the DV region (D442-V471) of 5-HT2AR was fused with TAT to obtain TAT-5HT2AR-DV; the CT region (K385-V471) of 5-HT2AR was fused with TAT to obtain TAT-5HT2AR-CT.

Mice were treated with TAT-5HT2AR-SV, TAT-5HT2AR-DV and TAT-5HT2AR-CT, and mice treated with TAT only were used as control (all treated by single intraperitoneal administration, 3nmol/g). 1 hour after the treatment, the mice used were analyzed according to the co-immunoprecipitation method in Example 1, and the results are shown in FIG. 5.

It can be seen from FIG. 5 that 5HT2AR-SV successfully destroyed the interaction of 5HT2AR/D1R in mouse hippocampus tissue.

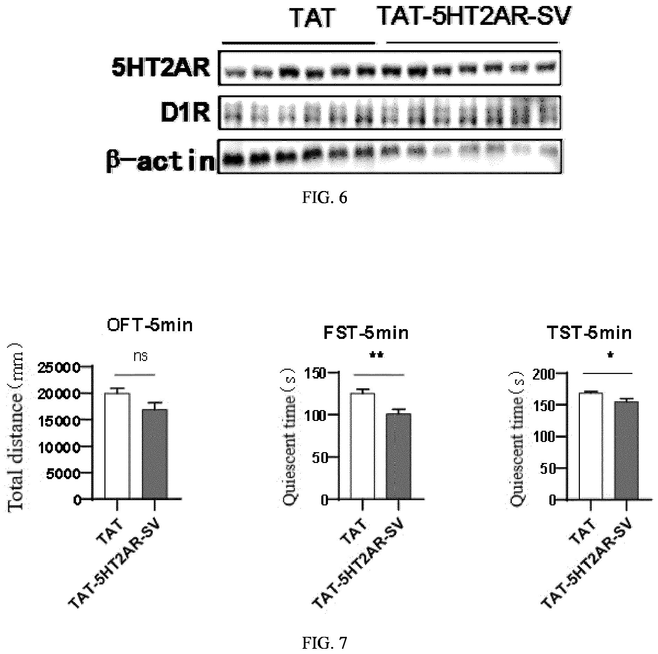

In addition, the hippocampus tissues of TAT-5HT2AR-SV and TAT-treated mice were directly analyzed by Western blot. As can be seen from FIG. 6, the expression of 5-HT2AR and D1R did not change significantly after treatment.

On the other hand, TAT-5HT2AR-DV and TAT-5HT2AR-CT also successfully destroyed the interaction of 5-HT2AR/D1R, and did not cause a significant change in the expression of 5-HT2AR and D1R.

Example 5. Evaluation of anti-depression-like effect of the polypeptide

To evaluate the anti-depression-like effect of the polypeptide of the present invention, mice treated with TAT-5HT2AR-SV, TAT-5HT2AR-DV, TAT-5HT2AR-CT (all treated by single intraperitoneal administration, 3nmol/g) were subjected to open field test (OFT), forced swimming test (FST), and tail suspension test (TST) 1 hour after treatment, with mice treated with TAT only as a control. Methods for the tests are as follows:

OFT: Mice were adapted to the experimental environment for 1 hour and were placed in a chamber of 45 × 45 × 30 cm. A 5-minute video was recorded to observe the movement activity of the mice. Total distance covered by the mice was measured and analyzed in millimeters.

FST: Mice were placed in a plexiglass cylinder (height 70 cm, diameter 30 cm) filled with water (with a water temperature of $23 \pm 1$ °C), and the height of water was over 30 cm. Mice were videotaped for 5 minutes and analyzed, and the length of time they remained quiescent was recorded. Mice are defined as quiescent when they float motionlessly in water or raise their noses above the water surface; horizontal movement over the whole cylinder is defined as swimming; vertical movement against the wall of the cylinder is defined as climbing.

TST: Mice were suspended with adhesive tape 40 cm above the floor in rectangular compartments (length 55 cm × width 20 cm × depth 11.5 cm). A video was recorded for 5 minutes, and the time they remained quiescent was recorded. The record and analysis were performed with EthoVisionXT software.

Some of the results are shown in FIG. 7. From the OFT results on the left, it can be seen that the total movement distance of mice treated with TAT-5HT2AR-SV was not significantly different from that of mice treated with TAT, indicating that the polypeptide had no effect on the movement ability of mice; in addition, from the FST results in the middle and TST results on the right, it can be seen that the quiescent time of TAT-5HT2AR-SV treated group was significantly reduced compared with the control group (Tat only). The results of the TAT-5HT2AR-DV treated group and TAT-5HT2AR-CT treated group were similar to those of the TAT-5HT2AR-SV treated group. The above confirms the anti-depression effect of the present invention.

Example 6. Evaluation of anti-depression ability of the polypeptide using a drug evaluation model

To further evaluate the ability of the polypeptide as an antidepressant, CRS, CMS and CSDS mouse models were constructed according to the method described in Example 2, respectively; 1 hour after construction, mouse models were treated with 10 μM TAT-5HT2AR-SV, TAT-5HT2AR-DV, TAT-5HT2AR-CT or TAT respectively (all treated by single intraperitoneal administration, 3nmol/g); then FST and TST were performed on CRS, and FST, TST and sucrose preference test (SPT) were performed on CMS and CSDS mice respectively.

Wherein the FST and TST operations are performed as described in Example 6; the specific operations of SPT are as follows:

SPT was performed using a two-bottle free-choice paradigm. Mice were adapted to 1% sucrose solution for 3 days and were randomly divided into groups. To assess their individual sucrose intake, mice were deprived of water and food for 24 hours within 3 days. On the next day, each mouse had free access to two bottles containing sucrose and water. After 2.5 hours, the position of bottles of water and sucrose were changed, and the total test time was 5 hours. Finally, the volumes of water and sucrose solution consumed were recorded and calculated according to the following formula (I):

$$SPT = \frac{\text{consumption of sucrose}}{\text{consumption of water and sucrose}} \times 100\% \qquad (I)$$

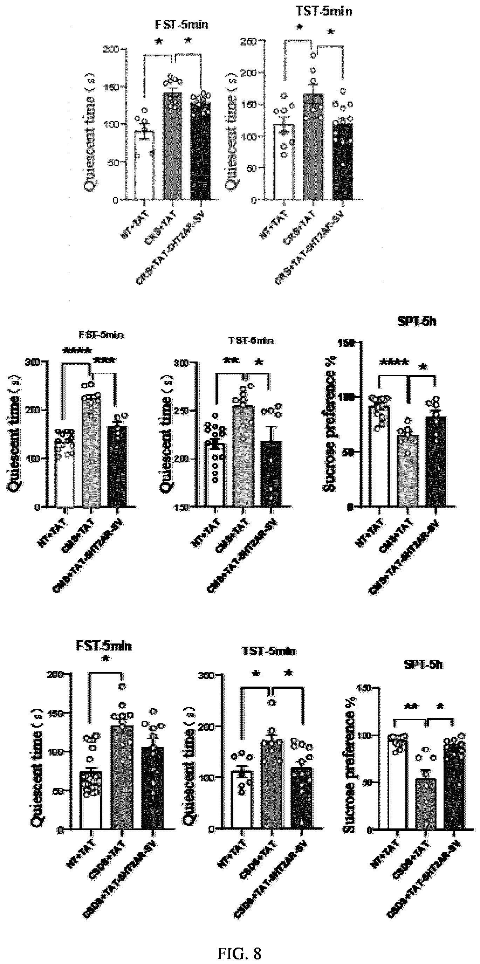

From the analysis results in FIG. 8, it can be seen that for all three drug evaluation models, mice treated with TAT-5HT2AR-SV significantly reduced the increase of stress-induced quiescent time (A to C, FST and TST) and significantly enhanced sucrose preference (A to C, SPT) compared with mice treated with TAT. The results of the TAT-5HT2AR-DV treated group and TAT-5HT2AR-CT treated group were similar to those of the TAT5HT2AR-SV treated group.

Example 7. Effectiveness of the polypeptide in disrupting complex interaction validated in an animal model

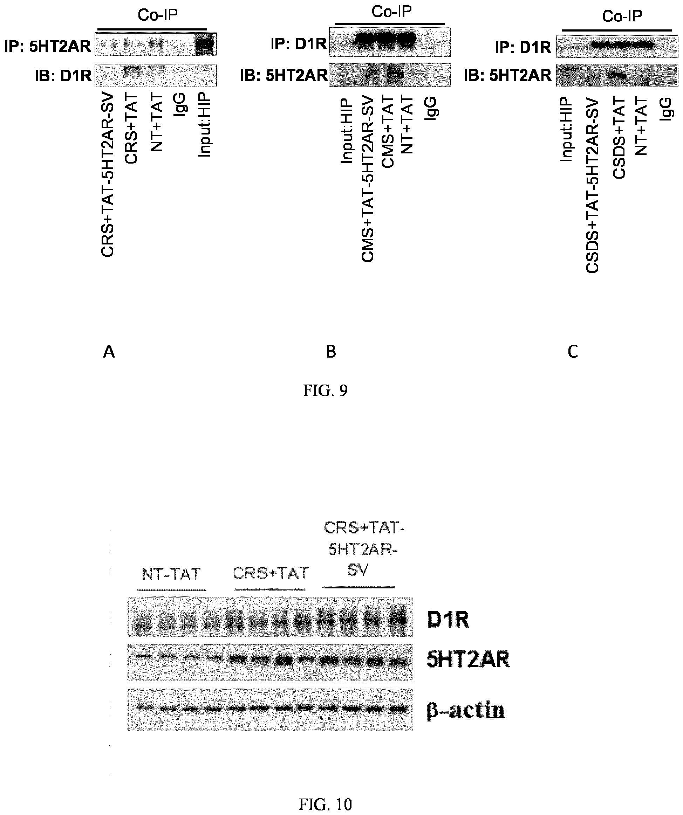

The treated CRS, CMS and CSDS mice of Example 6 (TAT-5HT2AR-SV, TAT-5HT2AR-DV, TAT-5HT2AR-CT and TAT) were analyzed according to the co-immunoprecipitation method of Example 1, and the Western blot results were shown in FIG. 9, respectively.

As expected, 5-HT2AR/D1R coupling levels increased significantly in all three models; TAT-5HT2AR-SV treated group significantly reduced the interaction between 5-HT2AR and D1R in hippocampus tissue of stressed mice compared with TAT treated group (FIG. 9 A to C).

Further, the hippocampus tissue of treated CRS mice was directly analyzed by Western blot to investigate the expression levels of D1R and 5-HT2AR, and the results are shown in FIG. 10. Interestingly, the levels of D1R and 5-HT2AR did not vary significantly between TAT-5HT2A-SV and TAT treated groups, indicating that the polypeptide of the present invention has a specificity for the coupling of D1R and 5-HT2AR.

In addition, CRS, CMS and CSDS mice treated with TAT-5HT2AR-DV or TAT-5HT2AR-CT were tested respectively. The results showed that TAT-5HT2AR-DV group and TAT-5HT2AR-CT group significantly reduced the interaction between 5-HT2AR and D1R in hippocampus tissue of stressed mice, but did not cause a significant change in D1R and 5-HT2AR levels.

Example 8. Evaluation of antianxiety-like effect of the polypeptide

To evaluate the antianxiety-like effect of the polypeptide of the present invention, mice treated with TAT-5HT2AR-SV, TAT-5HT2AR-DV, TAT-5HT2AR-CT (all treated by single intraperitoneal administration, 3nmol/g) were subjected to open field test (OFT) for measuring residence time in central area and elevated plus maze test (EPM) 1 hour after treatment, with TAT-treated mice as control. Methods for the tests are as follows:

OFT (for measuring the residence time in central area): open bottom surface was divided into 25 equal-area grids, with the middle 9 grids as the central area. 1 hour after treated with the polypeptide, mice were placed in the central area and recorded for 5 min, and the residence time of mice in the central area was recorded.

EPM: consists of two open arms (25cm × 8cm) and two closed arms (25cm × 8cm), with the intersection as the central area (8cm × 8cm), which is 40cm high from the ground. 1 hour after treated with the polypeptide, mice were placed in the central area facing the open arm, and were recorded for their free activities for 5 min. After each animal was tested, the device was wiped with 70% alcohol. The residence time at the open arm, the residence time at the closed arm, and the total movement distance of mice were recorded.

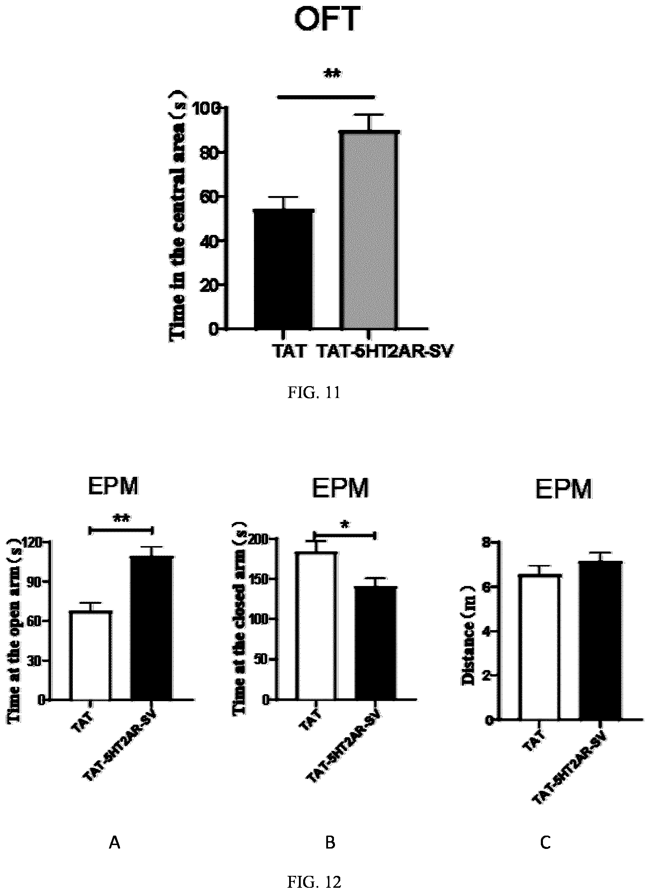

Some of the OFT results are shown in FIG. 11. TAT-5HT2A-SR polypeptide could significantly increase the residence time of mice in the central region; similarly, TAT-5HT2AR-DV polypeptide and TAT-5HT2AR-CT also significantly increased the residence time in the central region of mice ($P < 0.01$), indicating that they effectively reduced anxiety mood.

Some of the EPM results are shown in FIG. 12. TAT-5HT2A-SR polypeptide significantly increased residence time at the open arm (A) and decreased residence time at the closed arm (B), but did not affect the total movement ability of mice (C); similarly, TAT-5HT2AR-DV polypeptide and TAT-5HT2AR-CT significantly increased residence time at the open arm ($P < 0.01$) and decreased residence time at the closed arm ($P < 0.05$), but did not affect the total movement ability of mice. It is shown that the polypeptide of the present invention effectively reduced anxiety mood.

**Claims**

1. Use of a polypeptide in the preparation of a medicament for treating and/or preventing depression or anxiety disorder, wherein the polypeptide has an amino acid sequence as set forth in SEQ ID NO: 1.

2. The use according to claim 1, wherein the length of the polypeptide is 15 to 50aa.

3. The use according to claim 1, wherein the length of the polypeptide is 15 to 30aa.

4. The use according to any one of claims 1 to 3, wherein the polypeptide is derived from a 5-HT2A receptor.

5. The use according to claim 4, wherein the 5-HT2A receptor is derived from primate, preferably human.

6. Use of a nucleic acid molecule in the preparation of a medicament for treating and/or preventing depression or anxiety disorder, wherein the nucleic acid molecule encodes the polypeptide of any one of claims 1 to 5.

7. Use of an expression vector in the preparation of a medicament for treating and/or preventing depression or anxiety disorder, wherein the expression vector comprises the nucleic acid molecule of claim 6.

8. Use of a host cell in the preparation of a medicament for treating and/or preventing depression or anxiety disorder, wherein the host cell comprises the nucleic acid molecule of claim 6 or the expression vector of claim 7.

9. A complex comprising the polypeptide of any one of claims 1 to 5 and a carrier attached thereto for permeating the blood-brain barrier.

10. The complex according to claim 9, wherein the carrier for permeating the blood-brain barrier is one or more selected from the group consisting of: HIV-1 Tat protein, insulin, cationized albumin, monoclonal antibody against rat transferrin receptor, mouse-derived monoclonal antibody against human insulin receptor, Penetratin, transduction domain of Tat protein, Pep-1 peptide, $S4_{13}$-PV, Magainin 2 and Buforin 2.

**Co-IP**

IP: 5HT2AR

IgG  D1R  D2R  Input-HIP

FIG. 1

**Co-IP**

IP:D1R

IB: 5HT2AR

Input-HIP  IgG  NT  CRS  CMS  CSDS

FIG. 2

5HT2A

5HT2A-IL3(F255-V324)

5HT2A-CT(K385-V471)

5HT2A-KV(K385-V411)    5HT2A-DV(D442-V471)
5HT2A-NN(N412-N441)

5HT2A-DA(D442-A456)
5HT2A-SV(S457-V471)

5HT2A
1.416 bp

FIG. 3

FIG. 4

FIG. 5

FIG. 6

FIG. 7

FIG. 8

FIG. 9

FIG. 10

## OFT

FIG. 11

## EPM    EPM    EPM

A          B          C

FIG. 12

## INTERNATIONAL SEARCH REPORT

| International application No. |
|---|
| **PCT/CN2022/113892** |

| **A.** | **CLASSIFICATION OF SUBJECT MATTER** |
|---|---|
| | A61K 38/17(2006.01)i; A61K 47/64(2017.01)i; A61K 47/68(2017.01)i; A61P 25/22(2006.01)i; A61P 25/24(2006.01)i; C07K 14/705(2006.01)i; C12N 15/12(2006.01)i |

According to International Patent Classification (IPC) or to both national classification and IPC

| **B.** | **FIELDS SEARCHED** |
|---|---|

Minimum documentation searched (classification system followed by classification symbols)

A61K; A61P; C07K; C12N

Documentation searched other than minimum documentation to the extent that such documents are included in the fields searched

Electronic data base consulted during the international search (name of data base and, where practicable, search terms used)

CNABS, WPABS, ENTXT, CNTXT, DWPI, CATXT, USTXT, EPTXT, WOTXT, CNKI, WEB OF SCIENCE, PUBMED, ELSEVIER SCIENCE, 百度学术搜索, BAIDU SCHOLAR, GenBank, 中国专利序列检索系统, Chinese Patent Biological Sequence Retrieval System: 多肽, 5-HT2A受体, 治疗, 预防, 抑郁(症), 焦虑(症), 核酸分子, 表达载体, 宿主细胞, 复合体, 血脑屏障, HIV-1 Tat蛋白, 胰岛素, 阳离子化白蛋白, 抗大鼠转铁蛋白受体的单抗, 人胰岛素受体鼠源性单抗, Tat蛋白的转导结构域, Pep-1肽, Penetratin, S413-PV, Magainin 2, Buforin 2, polypeptide, 5-hydroxytryptamine, 5-HT2AR, Depression, Anxiety, Blood Brain Barrier, 基于SEQ ID NO: 1的序列检索, Sequence Retrieval based on SEQ ID NO:1

| **C.** | **DOCUMENTS CONSIDERED TO BE RELEVANT** | |
|---|---|---|
| Category* | Citation of document, with indication, where appropriate, of the relevant passages | Relevant to claim No. |
| PX | CN 113855784 A (SHENZHEN CHENYANG BIOTECHNOLOGY CO., LTD.) 31 December 2021 (2021-12-31) <br> see claims 1-10 | 1-10 |
| Y | CN 1920033 A (SHANDONG UNIVERSITY OF TRADITIONAL CHINESE MEDICINE) 28 February 2007 (2007-02-28) <br> see claims 1-3, and description, pages 1-2, sequence table | 1-10 |
| Y | WO 2018229744 A1 (GLYTECH LLC.) 20 December 2018 (2018-12-20) <br> see claims 1-13 | 1-10 |
| A | US 2018263976 A1 (GLYTECH LLC.) 20 September 2018 (2018-09-20) <br> see abstract | 1-10 |
| A | CN 101160127 A (ARENA PHARMACEUTICALS, INC.) 09 April 2008 (2008-04-09) <br> see abstract | 1-10 |

☑ Further documents are listed in the continuation of Box C.      ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 November 2022** | **28 November 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/113892**

**C.** **DOCUMENTS CONSIDERED TO BE RELEVANT**

| Category* | Citation of document, with indication, where appropriate, of the relevant passages | Relevant to claim No. |
|---|---|---|
| A | CN 101875961 A (ARENA PHARMACEUTICALS, INC.) 03 November 2010 (2010-11-03) see abstract | 1-10 |
| A | LIN, Jianyang et al. "Influence of 5-HTR2A genetic polymorphisms on the efficacy of antidepressants in the treatment of major depressive disorder: A meta-analysis" *Journal of Affective Disorders*, Vol. vol. 168, 15 October 2014 (2014-10-15), pp. 430-438, see abstract | 1-10 |
| Y | 蔡伯艳等 (CAI, Boyan et al.). "抑郁症与神经递质关系的研究进展 (Non-official translation: Research Progress on the Relationship between Depression and Neurotransmitters" *Journal of Liaoning University of Traditional Chinese Medicine*, Vol. 9, No. 5, 05 September 2007 (2007-09-05), pp. 51-52, see p. 51, left column | 1-10 |

Form PCT/ISA/210 (second sheet) (January 2015)

## INTERNATIONAL SEARCH REPORT

| International application No. |
|---|
| **PCT/CN2022/113892** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1] The actually submitted sequence table is an XML file of the standard ST.26.

| INTERNATIONAL SEARCH REPORT | | International application No. |
| :---: | :---: | :---: |
| Information on patent family members | | PCT/CN2022/113892 |

| Patent document cited in search report | | | Publication date (day/month/year) | Patent family member(s) | | | Publication date (day/month/year) |
| :--- | :--- | :--- | :--- | :--- | :--- | :--- | :--- |
| CN | 113855784 | A | 31 December 2021 | None | | | |
| CN | 1920033 | A | 28 February 2007 | None | | | |
| WO | 2018229744 | A1 | 20 December 2018 | KR | 20200027501 | A | 12 March 2020 |
| | | | | AU | 2018284335 | A1 | 30 January 2020 |
| | | | | CN | 110996948 | A | 10 April 2020 |
| | | | | EP | 3638234 | A1 | 22 April 2020 |
| | | | | CA | 3067162 | A1 | 20 December 2018 |
| | | | | IL | 271371 | A | 30 January 2020 |
| | | | | BR | 112019026449 | A2 | 11 August 2020 |
| | | | | ZA | 202000193 | B | 29 September 2021 |
| | | | | JP | 2020523345 | A | 06 August 2020 |
| | | | | RU | 2020100230 | A | 13 July 2021 |
| US | 2018263976 | A1 | 20 September 2018 | US | 2020206219 | A1 | 02 July 2020 |
| CN | 101160127 | A | 09 April 2008 | WO | 2006078610 | A1 | 27 July 2006 |
| | | | | AU | 2006206687 | A1 | 27 July 2006 |
| | | | | JP | 2008527042 | A | 24 July 2008 |
| | | | | EP | 1843762 | A1 | 17 October 2007 |
| | | | | CA | 2594563 | A1 | 27 July 2006 |
| | | | | US | 2008200530 | A1 | 21 August 2008 |
| CN | 101875961 | A | 03 November 2010 | JP | 2006528195 | A | 14 December 2006 |
| | | | | NO | 20060877 | L | 22 February 2006 |
| | | | | KR | 20060041260 | A | 11 May 2006 |
| | | | | CN | 101871931 | A | 27 October 2010 |
| | | | | HR | P20060100 | T3 | 31 March 2007 |
| | | | | DE | 602004000260 | D1 | 26 January 2006 |
| | | | | US | 2007072857 | A1 | 29 March 2007 |
| | | | | NZ | 544331 | A | 26 March 2010 |
| | | | | EP | 1558582 | A1 | 03 August 2005 |
| | | | | US | 2007078134 | A1 | 05 April 2007 |
| | | | | CN | 1826322 | A | 30 August 2006 |
| | | | | MY | 141862 | A | 16 July 2010 |
| | | | | TW | 200523253 | A | 16 July 2005 |
| | | | | US | 2020397755 | A1 | 24 December 2020 |
| | | | | US | 2018169070 | A1 | 21 June 2018 |
| | | | | US | 2015073141 | A1 | 12 March 2015 |
| | | | | RS | 20060035 | A | 07 August 2008 |
| | | | | IL | 172582 | D0 | 10 April 2006 |
| | | | | ES | 2258760 | T3 | 01 September 2006 |
| | | | | PT | 1558582 | E | 31 May 2006 |
| | | | | AR | 045069 | A1 | 12 October 2005 |
| | | | | AT | 313532 | T | 15 January 2006 |
| | | | | US | 2013237541 | A1 | 12 September 2013 |
| | | | | EC | SP066269 | A | 28 July 2006 |
| | | | | CA | 2533369 | A1 | 10 February 2005 |
| | | | | MX | PA06000795 | A | 23 August 2006 |
| | | | | JP | 2008143908 | A | 26 June 2008 |
| | | | | EA | 200600295 | A1 | 25 August 2006 |
| | | | | WO | 2005012254 | A1 | 10 February 2005 |
| | | | | TN | SN06017 | A1 | 03 October 2007 |

Form PCT/ISA/210 (patent family annex) (January 2015)

## INTERNATIONAL SEARCH REPORT
### Information on patent family members

International application No.

**PCT/CN2022/113892**

| Patent document cited in search report | Publication date (day/month/year) | Patent family member(s) | | | Publication date (day/month/year) |
|---|---|---|---|---|---|
| | | IS | 8241 | A | 19 January 2006 |
| | | JP | 2007039461 | A | 15 February 2007 |
| | | AU | 2004261582 | A1 | 10 February 2005 |
| | | BR | PI0412263 | A | 19 September 2006 |
| | | US | 2016374990 | A1 | 29 December 2016 |
| | | PL | 1558582 | T3 | 31 May 2006 |
| | | MA | 28066 | A1 | 01 August 2006 |
| | | DK | 1558582 | T3 | 08 May 2006 |
| | | US | 2005080124 | A1 | 14 April 2005 |

Form PCT/ISA/210 (patent family annex) (January 2015)